# EUROPÄISCHE PATENTANMELDUNG

(11) **EP 4 804 500 A2**
(43) Veröffentlichungstag der Anmeldung: **09.09.2026**
(21) Anmeldenummer: 26173373.7
(22) Anmeldetag: 23.08.2021
(51) Int. Cl.: H04L 67/568

(54) **VERFAHREN UND SYSTEM ZUR DATENÜBERTRAGUNG BEI BEATMUNGSGERÄTEN**

(30) Priorität: 15.09.2020 DE 102020123923
(62) Dokumentnummer(n) der früheren Anmeldung(en) nach Art. 76 EPÜ: 21000238.2 / 3 968 336
(71) Anmelder: Löwenstein Medical Technology S.A., 2557 Luxembourg (LU)
(72) Erfinder: Schwaibold, Matthias, 76228 Karlsruhe (DE)
(74) Vertreter: Löwenstein Medical IP

(57) **Zusammenfassung**

Verfahren zur Datenübertragung innerhalb eines Systems, wobei das System zumindest ein Beatmungsgerät und einen Server umfasst, wobei das Beatmungsgerät zumindest eine Eingabeeinheit und zumindest eine Kommunikationseinheit umfasst, wobei die Eingabeeinheit eingerichtet und ausgebildet ist, eingegebene Werte und Informationen dem System bereitzustellen und wobei die Kommunikationseinheit eingerichtet und ausgebildet ist, zumindest eine Verbindung zu zumindest einem Server herzustellen und Daten zwischen dem Server und dem Beatmungsgerät zu übertragen, also an den Server zu senden und vom Server zu empfangen, dadurch gekennzeichnet, dass die Datenübertragung durch den Server beendet wird, wobei während der Datenübertragung vom Server an das Beatmungsgerät Konfigurationsdaten gesendet, in denen medizinische Konfigurationsdaten, technische Konfigurationsdaten und Konfigurationsdaten zur Kommunikation enthalten sind, wobei die Konfigurationsdaten zur Kommunikation Einstellungen zur Datenmenge, die das Beatmungsgerät während der Datenübertragung an den Server senden soll, sowie auch zum Zeitintervall enthalten.

## Beschreibung

### Beschreibung

Die Erfindung betrifft ein Verfahren zur Datenübertragung innerhalb eines Systems umfassend zumindest ein Beatmungsgerät.

Moderne Beatmungsgeräte verfügen meist über mehrere Möglichkeiten der Anzeige oder Ausgabe von Daten bzw. Informationen bezüglich des Anwenders. So verfügt ein Beatmungsgerät in der Regel über ein Display, über welches die Informationen in einem gewissen Umfang direkt am Gerät dargestellt werden können. Darüber hinaus sind Beatmungsgeräte bekannt, welche über verschiedene Möglichkeiten zur Übertragung von Daten zwischen dem Beatmungsgerät und externen Geräten verfügen.

Im Stand der Technik sind diese Möglichkeiten in Form von Anschlüssen für Speichergeräte oder auch Verbindungen zu Servern bekannt, um die Daten vom Beatmungsgerät auf andere Geräte zu übertragen und dort anzeigen zu lassen oder (weiter) zu verarbeiten. Insbesondere eine Verbindung des Beatmungsgeräts mit einem Server ermöglicht die Fernüberwachung des Anwenders, das sogenannte Telemonitoring. Durch das Telemonitoring ist es möglich auch eine Mehrzahl an Beatmungsgeräten zu überwachen.

Die aus dem Stand der Technik bekannten Beatmungsgeräte sind zum Telemonitoring ständig mit Servern verbunden und tauschen dabei Daten und Informationen aus. Das führt dazu, dass auch zu Zeiten ohne die Notwendigkeit der Übertragung Daten übertragen und gespeichert werden. Damit einhergehend entstehen unnötige Energiekosten und gegebenenfalls auch Mobilfunkkosten bei mobil eingesetzten Geräten. Auch in Bezug auf den Datenschutz ist eine ständige Datenübertragung ohne Notwendigkeit kritisch zu sehen.

Aufgabe der vorliegenden Erfindung ist daher die Bereitstellung eines Systems zur Datenübertragung sowie ein entsprechendes Verfahren, welches den Energieverbrauch senken und die Überwachung mehrerer Beatmungsgeräte vereinfachen kann. Die Aufgabe wird durch ein Verfahren nach Anspruch 1 sowie durch die Vorrichtung nach Anspruch 27 gelöst.

Verfahren zur Datenübertragung innerhalb eines Systems, wobei das System zumindest ein Beatmungsgerät und einen Server umfasst, wobei das Beatmungsgerät zumindest eine Eingabeeinheit und zumindest eine Kommunikationseinheit umfasst, wobei die Eingabeeinheit eingerichtet und ausgebildet ist, eingegebene Werte und Informationen dem System bereitzustellen und wobei die Kommunikationseinheit eingerichtet und ausgebildet ist, zumindest eine Verbindung zu zumindest einem Server herzustellen und Daten zwischen dem Server und dem Beatmungsgerät zu übertragen, also an den Server zu senden und vom Server zu empfangen. Die Datenübertragung wird durch den Server beendet, wobei die Beendigung der Datenübertragung dadurch gekennzeichnet ist, dass vom Beatmungsgerät keine weiteren medizinischen Daten an den Server übertragen werden. Eine Beendigung der Datenübertragung durch den Server kann auch dadurch zustande kommen, dass der Server einen entsprechenden Befehl oder Aufforderung an das Beatmungsgerät sendet. Auch ist darunter zu verstehen, dass der Server eine Anforderung an Datenmenge und/oder Übertragungslänge an das Beatmungsgerät sendet und das Beatmungsgerät nach Übertragung der angeforderten Daten die Datenübertragung beendet. Das Beatmungsgerät entscheidet also nicht selbstständig, ob die Datenübertragung beendet wird, sondern nur nach Aufforderung und/oder Hinweisen (wie z.B. eine zu übertragende Datenmenge oder ein Zeitraum für die Datenübertragung) durch den Server.

In manchen Ausführungsformen des Verfahrens werden nicht-medizinische Daten unabhängig von der Datenübertragung zwischen dem Server und dem Beatmungsgerät übertragen.

In manchen Ausführungsformen des Verfahrens werden nach Beendigung der Datenübertragung durch den Server nicht-medizinische Daten zwischen Server und Beatmungsgerät übertragen.

In manchen Ausführungsformen des Verfahrens werden nach der Beendigung der Datenübertragung durch den Server, vom Server Daten an das Beatmungsgerät übertragen
In manchen Ausführungsformen des Verfahrens wird nach Beendigung der Datenübertragung zwischen dem Server und dem Beatmungsgerät die Verbindung zwischen Server und Beatmungsgerät getrennt.

In manchen Ausführungsformen des Verfahrens wird die Verbindung zwischen dem Beatmungsgerät und dem Server ausschließlich durch die Kommunikationseinheit hergestellt. Die Herstellung einer Verbindung zwischen Beatmungsgerät und Server kann beispielsweise auch durch eine entsprechende Eingabe an der Eingabeeinheit ausgelöst werden.

In manchen Ausführungsformen des Verfahrens verbindet sich die Kommunikationseinheit bei getrennter Verbindung in Zeitintervallen mit dem Server.

In manchen Ausführungsformen des Verfahrens wird die Verbindung nur dann hergestellt, wenn das Beatmungsgerät in einem verbindungsfähigen Modus ist.

In manchen Ausführungsformen des Verfahrens werden bei einer nach einem Zeitintervall neu aufgebauten Verbindung durch die Kommunikationseinheit des Beatmungsgerätes zum Server zunächst nur Geräteinformationen des Beatmungsgerätes an den Server übertragen.

In manchen Ausführungsformen des Verfahrens erkennt der Server anhand der übertragenen Geräteinformationen des Beatmungsgerätes das Beatmungsgerät und entscheidet, ob und welche Daten vom Beatmungsgerät an den Server übertragen werden sollen.

In manchen Ausführungsformen des Verfahrens sendet der Server vor Beendigung der Datenübertragung und/oder vor Trennen der Verbindung zumindest Daten bezüglich des Zeitintervalls, in dem sich das Beatmungsgerät mit dem Server verbindet.

In manchen Ausführungsformen des Verfahrens bestimmt das Beatmungsgerät das Zeitintervall für die nächste Verbindung mit dem Server anhand einer Analyse der zuvor übertragenen Daten automatisch.

In manchen Ausführungsformen des Verfahrens überträgt der Server Konfigurationsdaten zur Kommunikation an das Beatmungsgerät, wobei die Konfigurationsdaten zur Kommunikation Einstellungen zur Datenmenge, welche während der der Datenübertragung und/oder der Verbindung vom Beatmungsgerät an den Server gesendet werden sollen, sowie Einstellungen zu den Zeitabständen, in denen sich das Beatmungsgerät mit dem Server verbinden soll, umfassen.

In manchen Ausführungsformen des Verfahrens werden keine medizinischen Daten vom Beatmungsgerät zum Server übertragen, soweit diese Daten nicht vom Server angefordert werden.

In manchen Ausführungsformen des Verfahrens wird die Übertragung der Daten zwischen dem Beatmungsgerät und dem Server durch einen Aktionsbefehl vom Server an die Kommunikationseinheit begonnen. Dieser Aktionsbefehl übermittelt an die Kommunikationseinheit bzw. das Beatmungsgerät zum Beispiel die Anforderung einer Datenübertragung.

In manchen Ausführungsformen des Verfahrens erzeugen die Kommunikationseinheit und/oder der Server ein Alarmsignal oder lösen ein Alarmsignal aus, wenn eine bestimmte Anzahl von nicht in den vorgegebenen Zeitintervallen erfolgten oder nicht erfolgreichen Verbindungen der Kommunikationseinheit zum Server registriert werden.

In manchen Ausführungsformen des Verfahrens wird die Datenübertragung der Kommunikationseinheit zum Server durch den Server automatisch anhand einer Analyse der zuvor vom Beatmungsgerät übertragenen medizinischen Daten beendet.

In manchen Ausführungsformen des Verfahrens wird die Verbindung der Kommunikationseinheit zum Server durch den Server automatisch anhand einer Analyse der zuvor übertragenen Daten getrennt.

In manchen Ausführungsformen des Verfahrens wird die Datenübertragung der Kommunikationseinheit zum Server durch den Server nach einer Eingabe am Server beendet.

In manchen Ausführungsformen des Verfahrens wird die Verbindung der Kommunikationseinheit zum Server durch den Server nach einer Eingabe am Server getrennt.

In manchen Ausführungsformen des Verfahrens werden Daten von dem Beatmungsgerät aufgezeichnet und bis zur nächsten Verbindung mit dem Server gespeichert. In manchen Ausführungsformen werden die aufgezeichneten Daten auch nach einer Verbindung mit dem Server und/oder einer Übertragung der gespeicherten Daten an den Server gespeichert.

In manchen Ausführungsformen des Verfahrens stellt das Beatmungsgerät bei getrennter Verbindung zum Server anhand einer Analyse der aufgezeichneten Daten eine Verbindung zu dem Server her und initiiert eine Datenübertragung.

In manchen Ausführungsformen des Verfahrens wertet das Beatmungsgerät die gespeicherten Daten aus und/oder fasst diese zusammen. Diese ausgewerteten beziehungsweise zusammengefassten Daten werden im Verlauf einer neuen Übertragung von Daten an den Server übertragen.

In manchen Ausführungsformen des Verfahrens wertet das Beatmungsgerät die gespeicherten Daten aus und/oder fasst diese zusammen. Diese ausgewerteten beziehungsweise zusammengefassten Daten werden im Verlauf einer neuen Übertragung von Daten an den Server übertragen, soweit sie vom Server angefordert werden.

In manchen Ausführungsformen des Verfahrens umfassen die Konfigurationsdaten zur Kommunikation Einstellungen, ob Daten mit hohem Detailgrad übertragen werden sollen.

In manchen Ausführungsformen des Verfahrens umfassen die Konfigurationsdaten zur Kommunikation Einstellung, ob ausschließlich bewertete und/oder zusammengefasste Daten übertragen werden sollen.

In manchen Ausführungsformen des Verfahrens verbindet sich das Beatmungsgerät in gewissen Zeitabständen mit dem Server, und es erfolgt eine Datenübertragung zwischen dem Server und dem Beatmungsgerät.

In manchen Ausführungsformen des Verfahrens wird die Übertragung von medizinischen Daten verhindert wird, nachdem die Datenübertragung durch den Server beendet wurde.

Die beanspruchte Vorrichtung betrifft ein Beatmungsgerät, welches in dem beschriebenen, erfindungsgemäßen Verfahren eingesetzt wird.

Es ist darauf hinzuweisen, dass die in den Ansprüchen einzeln aufgeführten Merkmale in beliebiger, technisch sinnvoller Weise miteinander kombiniert werden können und weitere Ausgestaltungen der Erfindung aufzeigen. Die Beschreibung charakterisiert und spezifiziert die Erfindung insbesondere im Zusammenhang mit den Figuren zusätzlich.

Es sei ferner darauf hingewiesen, dass eine hierin verwendete, zwischen zwei Merkmalen stehende und diese miteinander verknüpfende Konjunktion "und/oder" stets so auszulegen ist, dass in einer ersten Ausgestaltung des erfindungsgemäßen Gegenstands lediglich das erste Merkmal vorhanden sein kann, in einer zweiten Ausgestaltung lediglich das zweite Merkmal vorhanden sein kann und in einer dritten Ausgestaltung sowohl das erste als auch das zweite Merkmal vorhanden sein können.

Unter einem Beatmungsgerät ist jedwedes Gerät zu verstehen, welches einen Anwender oder Patienten bei der natürlichen Atmung unterstützt, die Beatmung des Anwenders bzw. Patienten übernimmt und/oder zur Atemtherapie dient und/oder anderweitig die Atmung des Anwenders bzw. Patienten beeinflusst. Darunter fallen zum Beispiel, aber nicht ausschließend, CPAPsowie BiPAP-Geräte, Narkose- bzw. Anästhesiegeräte, Atemtherapiegeräte, (klinische, außerklinische oder Notfall-) Beatmungsgeräte, Highflow-Therapiegeräte und Hustenmaschinen. Beatmungsgeräte können auch als Diagnosegeräte für die Beatmung verstanden werden. Diagnosegeräte können dabei allgemein zur Erfassung von medizinischen Parametern eines Patienten dienen. Darunter fallen auch Geräte, welche medizinische Parameter von Patienten in Kombination mit oder ausschließlich die Atmung betreffend, erfassen und optional verarbeiten können.

Eine Schnittstelle ist im weitesten Sinne als jede Art von Einrichtung zur Verbindung - unabhängig der Verbindungsart - zwischen dem Beatmungsgerät und einer Gegenstelle oder einer Person zu verstehen. Eine Schnittstelle für eine Verbindung zwischen dem Beatmungsgerät und einer Person kann dabei zum Beispiel eine Benutzerschnittstelle sein, welche die Interaktion zwischen Person und Beatmungsgerät direkt am Beatmungsgerät ermöglicht.

Als Patienten-Interface kann, soweit nicht ausdrücklich anders beschrieben, jeglicher Teil oder verbundene Peripheriegeräte des Beatmungsgerätes verstanden werden, welche zur Interaktion, insbesondere zu Therapie- oder Diagnosezwecken, mit einem Patienten gedacht ist. Insbesondere kann ein Patienten-Interface als Maske eines Beatmungsgerätes bzw. eine mit dem Beatmungsgerät verbundene Maske verstanden werden. Diese Maske kann eine Full-Face Maske, also Nase und Mund umschließende, oder eine Nasenmaske, also eine nur die Nase umschließende Maske, sein. Auch Trachealtuben und sogenannte Nasenbrillen können als Maske eingesetzt werden.

Wurde beispielsweise die Datenübertragung zwischen Beatmungsgerät und Server beendet und damit einhergehend auch die Verbindung zwischen Beatmungsgerät und Server getrennt, so kann für das Beatmungsgerät eingestellt werden das bei getrennter Verbindung zwischen Beatmungsgerät und Server in einem bestimmten Zeitintervall, welches zum Beispiel am Beatmungsgerät festgelegt wird und/oder durch den Server vorgegeben wird, durch die Kommunikationseinheit eine Verbindung zum Server hergestellt wird beziehungsweise ein Verbindungsversuch erfolgt. Die Verbindung wird in manchen Ausführungsformen nur dann hergestellt, wenn das Beatmungsgerät zu einer Verbindung und Datenübertragung in der Lage ist. Dazu sollte zum Beispiel zumindest eine Stromversorgung sowie eine Datenverbindung (beispielsweise Mobilfunk oder Internetverbindung über LAN/WLAN) vorhanden sein. Zum Teil ist während einer laufenden Schlaftherapie eine aktive drahtlose Netzwerkverbindung (zum Beispiel Mobilfunk, WLAN/WiFi) nicht erwünscht. Ist eine kabelgebundene Netzwerkverbindung beziehungsweise Internetverbindung nicht verfügbar, könnte in diesem Fall keine Verbindung vom Beatmungsgerät zum Server hergestellt werden.

Die Verbindung und eine Datenübertragung zwischen Beatmungsgerät und dem Server wird regelmäßig hergestellt. Dazu stellt das Beatmungsgerät eine Verbindung zum Server her, sobald ein vorher definiertes Zeitintervall abgelaufen ist und das Beatmungsgerät dazu in der Lage ist, also zum Beispiel eine Stromversorgung und Datenverbindung verfügbar sind. Die Herstellung einer Verbindung von Beatmungsgerät und Server kann alternativ auch manuell durch einen Benutzer am Beatmungsgerät oder einen Benutzer des Servers ausgelöst werden.

Während der Datenübertragung werden vom Server an das Therapiegerät Konfigurationsdaten gesendet, in denen medizinische Konfigurationsdaten (zum Beispiel therapeutische Einstellwerte), technische Konfigurationsdaten (zum Beispiel Firmware-Update) und Konfigurationsdaten zur Kommunikation enthalten sind. Die Konfigurationsdaten zur Kommunikation enthalten Einstellungen zur Datenmenge, die das Beatmungsgerät während der Datenübertragung an den Server senden soll, sowie bevorzugt auch zum Zeitintervall. Mit der Konfiguration zur Kommunikation kann weiter auch zum Beispiel eingestellt werden, wie detailliert die medizinischen und technischen Daten des Beatmungsgerätes an den Server übermittelt werden sollen.

Während der Datenübertragung werden vom Therapiegerät an den Server technische Daten (zum Beispiel Versionsstand, Einstellungen, Füllgrad/Füllstand bzw. Abnutzung von Verbrauchsmaterialen, Auftreten technischer Fehler etc.) und medizinische Daten (Nutzungszeiten des Beatmungsgerätes, Wirksamkeit des Beatmungsgerätes/der Therapie, korrekte Anwendung des Beatmungsgerätes, diagnostische Daten aus dem Beatmungsgerät, Druck, Fluss, Atemfrequenz, Volumina, O₂/CO₂-Sättigung/Gehalt der (Aus-)Atemluft, etc.) übermittelt.

Über die Konfigurationsdaten zur Kommunikation kann der Server, bzw. ein Benutzer des Servers, aus der Ferne insbesondere einstellen, ob, mit welchem Zeitintervall und mit welchem Detailgrad medizinische Daten vom Beatmungsgerät an den Server gesendet werden sollen. Die medizinischen Daten können in unterschiedlichen Detailgraden gesendet werden. Den Detailgrad der Übermittlung kann der Server vorgeben. Den Detailgrad der Übermittlung kann auch ein Benutzer des Servers aus der Ferne einstellen. In manchen Ausführungsformen kann auch das Therapiegerät selbst den Detailgrad vorgeben. Die Daten können beispielsweise Messwerte und/oder Ergebniswerte sein. Die Daten können auch in Form von Signalkurven übermittelt werden. Die Daten bzw. Werte können als Rohdaten übermittelt werden oder als Kenngrößen wie beispielsweise Mittelwerte, Mediane oder dergleichen. Die Auflösung der Daten kann ebenfalls vom Server vorgegeben werden. Die Daten können mit niedriger, mittlerer oder hoher Auflösung gesendet werden.

Auch können die Konfigurationsdaten zur Kommunikation Einstellungen darüber enthalten, ob eine dauerhafte Datenübertragung, also ein ständiges Senden von aufgenommenen Daten, oder eine Übertragung von Datenpaketen, zum Beispiel zusammengefasste medizinische Daten, übermittelt werden sollen. Bezüglich einer dauerhaften Datenübertragung kann beispielsweise zusätzlich eingestellt werden, mit welcher Frequenz die Daten übertragen werden, also ob eine Liveübertragung oder eine geringere Frequenz (zum Beispiel eine Übertragung alle 1 bis 60 Minuten) erfolgen soll.

Dabei können bei jeder Datenübertragung beziehungsweise Verbindung neue Konfigurationsdaten zur Kommunikation vom Server an das Beatmungsgerät gesendet werden, welches diese bei aktuellen und/oder zukünftigen Verbindungen beziehungsweise Datenübertragungen anwendet.

In einigen Ausführungsformen des Verfahrens sendet das Beatmungsgerät bei jeder neu hergestellten Verbindung zum Server zunächst Geräteinformationen, welche Informationen wie Gerätetyp, Seriennummer, Firmware-Version etc. enthalten, an den Server. Anhand dieser Informationen erkennt der Server zunächst das Gerät und kann daraufhin individuell für dieses Gerät entscheiden, ob und welche Datenmenge vom Beatmungsgerät an den Server gesendet werden soll. Diese Entscheidung wird zum Beispiel als Konfiguration zur Kommunikation vom Server an das Beatmungsgerät gesendet. Basierend auf dieser Konfiguration zur Kommunikation beginnt das Beatmungsgerät gegebenenfalls mit der Datenübertragung. Auch entscheidet der Server ob und welche weiteren Daten und/oder Konfigurationen, wie zum Beispiel technische oder medizinische Konfigurationen, an das Beatmungsgerät gesendet werden. Zum Beispiel kann der Server anhand der Geräteinformationen des Beatmungsgeräts entscheiden, dass eine neue Firmware-Version an das Beatmungsgerät gesendet werden soll.

In manchen Ausführungsformen des Verfahrens bekommt das Beatmungsgerät anhand der Konfiguration zur Kommunikation auch das Zeitintervall, nach dem es sich erneut mit dem Server verbinden soll, sobald die Datenübertragung abgeschlossen ist. Alternativ kann das Beatmungsgerät auch dazu eingerichtet sein, das Zeitintervall anhand der übertragenen Daten selbst zu bestimmen. Wurden zum Beispiel gerade medizinische Daten abgefragt und vom Beatmungsgerät an den Server gesendet, dann erfolgt die nächste Verbindung vom Beatmungsgerät zum Server nach einem Zeitintervall von zum Beispiel 24 Stunden. Wurden keine medizinischen Daten angefordert, kann das Zeitintervall beispielsweise auch 3 oder 5 oder 7 Tage betragen.

Nach Ablauf des Zeitintervalls zur nächsten Verbindung des Beatmungsgeräts mit dem Server, wird sowohl vom Beatmungsgerät als auch vom Server registriert, ob eine Verbindung erfolgreich hergestellt wurde. Bei manchen Ausführungsformen des Verfahrens wird auf Seiten des Servers beispielsweise die Anzahl der nicht erfolgten Verbindungen gezählt und ab einer bestimmten Anzahl an ausgebliebenen Verbindungen eine Alarmmeldung generiert, welche über den Server ausgegeben und einem Nutzer des Servers angezeigt wird. Alternativ oder ergänzend kann der Server auch so eingestellt sein, dass eine Verbindung zum Beatmungsgerät durch den Server angestrebt wird, sollte eine Anzahl an nicht erfolgten Verbindungen registriert werden. Die Anzahl an nicht erfolgten Verbindungen zwischen Beatmungsgerät und Server, welche zur Generierung einer Alarmmeldung führt ist unter anderem abhängig von dem vorher eingestellten Zeitintervall und der letzten Datenübertragung. Ist beispielsweise das eingestellte Zeitintervall 24 Stunden, so kann nach 2 nicht erfolgten Verbindungen (also 48 Stunden) bereits eine Alarmmeldung durch den Server generiert werden. Auch eine Schwelle von nur einer nicht erfolgten Verbindung oder 3 und mehr nicht erfolgten Verbindungen sind denkbar und können sinnvoll sein. War die letzte Datenübertragung eine dauerhafte, also "live"-Übertragung bzw. eine Übertragung von Daten in kurzen Zeitabständen (Frequenz von einer Datenübertragung alle 1 bis 360 Minuten), so kann es sinnvoll sein, die Anzahl der nicht erfolgten Verbindungen, welche zu einer Alarmmeldung führen, höher zu wählen. Zum Beispiel kann auch hier die Anzahl gewählt werden, welche äquivalent zu 24 Stunden oder 2 Tagen oder mehr ist. Zum Beispiel würde das bei einer Frequenz von einer Verbindung bzw. Datenübertragung alle 60 Minuten bedeuten, dass 24 nicht erfolgte Verbindungen vom Server registriert werden, bevor eine Alarmmeldung durch den Server generiert wird. Gleiche Regeln können auch für das Beatmungsgerät gelten. Zusätzlich kann das Beatmungsgerät auch eingerichtet sein, nach einer erfolglosen Verbindung zum Server die Zeitintervalle, nach denen eine Verbindung erfolgen soll automatisch zu verkürzen. Soll beispielsweise eine Verbindung nach 24 Stunden hergestellt werden, dies aber aus nicht näher bestimmten Gründen nicht möglich sein, so kann das Zeitintervall zunächst auf 1 bis 12 Stunden verkürzt werden. Kann nach einer bestimmten Anzahl von Verbindungsversuchen noch immer keine Verbindung zum Server hergestellt werden, so wird beispielsweise eine Alarmmeldung generiert und dem Nutzer am Beatmungsgerät angezeigt. Die Anzahl der nicht erfolgreichen Verbindungen durch das Beatmungsgerät, welche zu einer Alarmmeldung führen, kann beispielsweise 1 bis 72 erfolglose Verbindungen betragen. Die Alarmmeldung kann beispielsweise neben dem Hinweis, dass die letzte oder die letzten Verbindungen nicht erfolgt sind, auch Hinweise darauf enthalten, dass eventuell Einstellungen am Gerät geändert werden müssen und/oder der Service kontaktiert werden sollte.

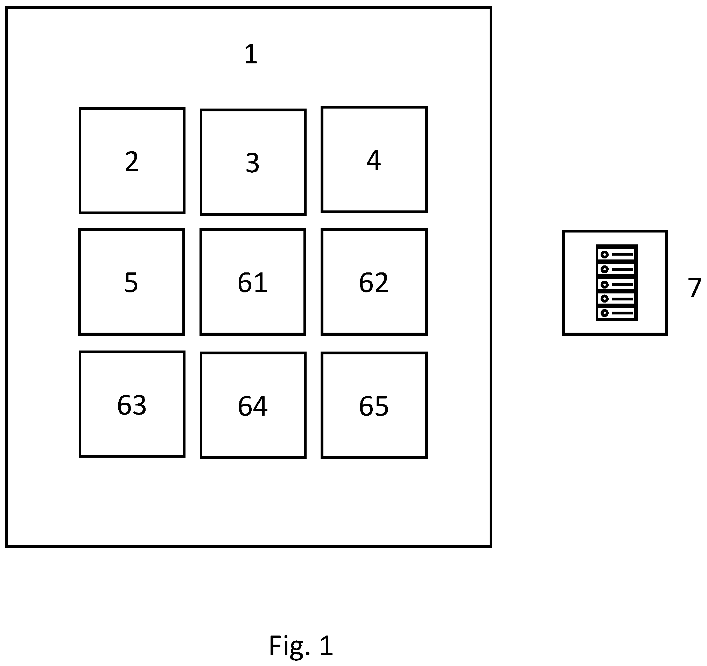

Im Folgenden wird die Erfindung anhand der Figuren 1 bis 3 über beispielhafte Ausführungsformen näher erläutert.

Die Figur 1 zeigt dabei den beispielhaften, schematischen Aufbau des Systems mit dem Beatmungsgerät 1 und dem Server 7.

Das Beatmungsgerät umfasst beispielsweise eine Eingabeeinheit 2, eine Kommunikationseinheit 3, eine Steuereinheit 4, eine Benutzerschnittstelle 5, eine Gebläse- und/oder Ventileinheit 61, eine Sensoreinheit 62, eine Aufbereitungseinheit 63, eine Speichereinheit 64 sowie eine Überwachungseinheit 65.

Die Gebläse- und/oder Ventileinheit 61 ist ausgebildet um einen Atemgasluftstrom zur Beatmung bzw. Therapie eines Patienten zu erzeugen und gegebenenfalls in Richtung des Patienten zu fördern.

Die Sensoreinheit 62 ist eingerichtet, Messwerte, insbesondere Parameter, die mit einem Atemfluss, einem Atemvolumen, einer Atemfrequenz, einer Einatmungs- und Ausatmungsdauer, einer Atemkontur, einer Leckage oder einem Therapiedruck in Zusammenhang stehen, zu erfassen. Optional kann die Sensoreinheit 62 zusätzliche Messungen von Bestandteilen oder Temperatur des Atemgases oder des Blutes vornehmen. Die Sensoreinheit 62 übermittelt die erfassten Messwerte an die Aufbereitungseinheit 63.

Die Aufbereitungseinheit 63 kann die erfassten Messwerte aufbereiten. Beispielsweise kann die Aufbereitungseinheit 63 eine Glättung, eine Artefaktbereinigung oder ein Downsampling der Messwerte durchführen. In manchen Ausführungsformen ist die Aufbereitungseinheit 63 auch als kombinierte Aufbereitungs-, Berechnungs- und Erkennungseinheit ausgebildet, alternativ oder ergänzend können diese Einheiten auch als separate Einheiten ausgebildet sein. Die Berechnungseinheit berechnet aus den durch die Sensoreinheit erfassten und durch die Aufbereitungseinheit aufbereiteten Messwerten Signale und/oder Kenngrößen, wie beispielsweise einen Mittelwert, einen Median, ein Perzentil, eine Ableitung, eine Häufigkeitsverteilung, eine Dauer oder einen Anteil eines Über- oder Unterschreitens von Schwellenwerten. Die Erkennungseinheit ist eingerichtet, Ereignisse/Zustände wie beispielsweise Alarme, Atemaussetzer, Artefakte, Hustenstöße, Sauerstoff(ent)sättigungen, asynchrone zwischen Gerät und Anwender, Einatmen, Ausatmen oder mandatorische Atemzüge zu erkennen.

Die Speichereinheit 64, welche beispielsweise auch als Zwischenspeichereinheit ausgebildet sein kann, speichert unter anderem die durch die Sensoreinheit 62 erfassten Werte/Parameter und/oder die durch die Aufbereitungseinheit 63 aufbereiteten Werte, Daten und/oder Informationen, beziehungsweise speichert diese zumindest zwischen. Eine Zwischenspeicherung bedeutet zum Beispiel, dass die Werte, Daten und/oder Informationen bis zu einer Übertragung gespeichert und danach zum Beispiel gelöscht oder zum Überschreiben freigegeben werden.

Die Überwachungseinheit 65 erfasst zum Beispiel technische Probleme des Beatmungsgerätes 1. Technische Probleme können beispielsweise ein niedriger Akkustand, Fehler in der Elektronik, ein defekter Akku, ein defektes Bauteil, ein Stromausfall, ein nicht korrekt funktionierendes Zubehörteil, ein unplausibler Messwert oder ein Verlassen eines erlaubten Temperaturbereichs sein. Die Überwachungseinheit kann bei einem erkannten technischen Problem einen Alarm auf dem Beatmungsgerät 1 über eine der Schnittstellen anzeigen oder übermitteln. In manchen Ausführungsformen können diese technischen Probleme zum Teil auch als schwerwiegende Fehler angesehen werden, welche, außer einer entsprechenden Fehlermeldung am Display (Benutzerschnittstelle), keine Datenübertragungen zulässt.

Zusätzlich umfasst das Beatmungsgerät 1 beispielsweise hier nicht gezeigte Anschlüsse um zum Beispiel ein Patienten-Interface über einen Schlauch mit dem Beatmungsgerät 1 zu verbinden.

Die Eingabeeinheit 2 ist beispielsweise so eingerichtet und ausgebildet, dass sie dem System bzw. insbesondere dem Beatmungsgerät 1 Daten und/oder Informationen zur Verfügung stellt. Die Eingabeeinheit 2 ist dabei mit der Kommunikationseinheit 3 sowie der Benutzerschnittstelle 5 so verbunden, dass die durch ebendiese Einheiten 3,5 eingegebenen Daten und/oder Informationen dem System bzw. dem Beatmungsgerät 1 zur Verfügung gestellt werden.

Die Kommunikationseinheit 3 dient beispielsweise primär zur Verbindung des Beatmungsgeräte 1 zum Server 7.

Die Steuereinheit 4 des Beatmungsgerätes 1 ist beispielhaft dazu eingerichtet, zumindest die Gebläse- bzw. Ventileinheit 61 zu steuern. In manchen beispielhaften Ausführungsformen ist die Steuereinheit 4 auch dazu eingerichtet, die Kommunikationseinheit 3 und/oder andere bzw. alle weiteren Bestandteile des Beatmungsgerätes 1 zu steuern. In manchen Ausführungsformen kann auch eine Vielzahl von Steuereinheiten eingesetzt sein. In der Regel steuert die Steuereinheit 4 die Gebläse- bzw. Ventileinheit 61 auf nach Vorgabe der in der Speichereinheit 64 hinterlegten Konfigurationen sowie durch die Sensoreinheit 62 ermittelten und eventuell durch die Aufbereitungseinheit 63 aufbereiteten/ausgewerteten Daten. Überschreiten die aufbereiteten/ausgewerteten Daten beispielsweise Schwellenwerte bei Atemdrücken, Gasflüssen und/oder Atemfrequenzen und wird dies durch die Aufbereitungseinheit 63 aus den aufgezeichneten Werten der Sensoreinheit 62 erkannt, so regelt die Steuereinheit 4 entsprechend die Gebläse- bzw. Ventileinheit 61.

Die Benutzerschnittstelle 5 ist zum Beispiel eine Schnittstelle um dem Anwender/Nutzer/Patienten am Beatmungsgerät die Möglichkeit zu geben Einstellungen, Konfigurationen und Eingaben zu tätigen und sich gegebenenfalls auch Meldungen (z.B. Alarmmeldungen), Informationen und Daten (z.B. medizinische und technische Daten) anzeigen zu lassen. Die Benutzerschnittstelle 5 kann beispielsweise als berührungsempfindlicher Bildschirm ("Touchscreen") ausgeführt sein, welcher gleichzeitig Eingabe als auch Anzeige ermöglicht. Auch kann die Benutzerschnittstelle als Bildschirm mit zusätzlichen Knöpfen, Schaltern und/oder Drehschaltern zur Eingabe ausgeführt sein. In manchen beispielhaften Ausführungsformen besteht die Benutzerschnittstelle lediglich aus einem oder mehreren Bedienelementen für die rudimentäre Bedienung (z.B. Ein-/Ausschalten) sowie optional Leuchtmittel zur Statusanzeige. Die Benutzerschnittstelle 5 ist beispielhaft auch dazu eingerichtet, eine Aufforderung zur Verbindung des Beatmungsgerätes 1 über die Kommunikationseinheit 3 zum Server 7 einzugeben. In manchen beispielhaften Ausführungsformen ist die Benutzerschnittstelle 5 eine Schnittstelle zur Verbindung mit externen Anzeige- und Ausgabegeräten, wie zum Beispiel einer Tastatur und einem externen Bildschirm.

Der Server 7 ist unteranderem dazu eingerichtet, dass eine Verbindung zwischen der Kommunikationseinheit 3 des Beatmungsgerätes 1 und dem Server 7 hergestellt werden kann. In manchen beispielhaften Ausführungsformen verfügt der Server 7 zu dem auch über einen angeschlossenen Computer oder ist direkt mit zumindest einer Anzeigeeinheit sowie einem Eingabegerät (z.B. Tastatur) verbunden. Weiter ist der Server 7 beispielsweise auch dazu ausgebildet, vom Beatmungsgerät 1 empfangene Daten und/oder Informationen zu speichern, auszuwerten, verarbeiten, weiterzuleiten und/oder zu interpretieren. Auch können durch den Server 7 Informationen, Daten und/oder Konfigurationen an das Beatmungsgerät 1 gesendet werden. Über den Computer und/oder die Eingabeeinheit können Eingaben, wie z.B. Daten und Informationen und auch Konfigurationen, am Server 7 durch einen Nutzer/Anwender eingegeben werden, welche beispielsweise dann an das Beatmungsgerät 1 weitergeleitet werden.

Zur näheren beispielhaften Erläuterung des Verfahrens wird angenommen, dass bereits eine Verbindung zwischen dem Beatmungsgerät 1 und dem Server 7 besteht. Über die Kommunikationseinheit 3 werden Daten vom Beatmungsgerät 1 an den Server 7 gesendet. Diese Daten enthalten beispielsweise technische und medizinische Daten des Beatmungsgerätes. Technische Daten können zum Beispiel der Versionsstand der Firmware, Einstellungen des Beatmungsgerätes, Füllgrad/Füllstand und/oder Abnutzung von Verbrauchsmaterialien, Auftreten technischer Fehler, etc. sein. In manchen Ausführungsformen werden diese technischen Daten insbesondere durch die Überwachungseinheit 65 ermittelt. Medizinische Daten umfassen beispielsweise die Nutzungszeiten und -dauer des Beatmungsgerätes, die Wirksamkeit des Beatmungsgerätes, die korrekte Anwendung, sowie diagnostische und/oder therapiebezogene Daten, wie zum Beispiel Beatmungsdrücke, Atemfrequenz, Fluss-Daten, Temperatur des Patienten und/oder der Atemluft. Zudem können auch noch weitere, hier nicht genannte, medizinische Daten vom Beatmungsgerät 1 an den Server 7 übertragen werden.

Sind alle durch den Server 7 angeforderten Daten übertragen worden und sollen keine weiteren Daten und/oder Konfigurationen vom Server 7 an das Beatmungsgerät 1 gesendet werden, kann der Server 7 oder ein Benutzer/Anwender am Server 7 entscheiden, die Datenübertragung zumindest der medizinischen Daten zu beenden und daraufhin gegebenenfalls auch die Verbindung zum Beatmungsgerät 1 zu trennen. Bevor der Server 7 die Verbindung zum Beatmungsgerät 1 trennt, werden zumindest Konfigurationsdaten zur Kommunikation übermittelt, welche zumindest das Zeitintervall vorgeben, nach welchem das Beatmungsgerät 1 die nächste Verbindung zum Server 7 herstellen soll. In einer alternativen oder zusätzlichen Ausführungsform kann auch das Beatmungsgerät 1 automatisch, beispielsweise anhand einer Analyse der medizinischen Daten durch die Aufbereitungseinheit, oder per Eingabe über die Benutzerschnittstelle 5 bestimmen, wann es die nächste Verbindung zum Server 7 herstellt. Der Server 7 und/oder auch das Beatmungsgerät 1 entscheidet zum Beispiel basierend auf den während der aktuellen Verbindung vom Beatmungsgerät 1 an den Server 7 gesendeten Daten oder Datenmenge bzw. der Übertragungszeit über das Zeitintervall bis zur nächsten Verbindung. Bei einer dauerhaften Datenübertragung von zumindest medizinischen Daten, also einem "Live"-Monitoring des Beatmungsgerätes 1, kann das Zeitintervall bis zur nächsten Verbindung zum Beispiel im Bereich von 1 bis 360 Minuten oder auch bis zu 7 Tage liegen. Zum Beispiel kann der Server 7 anhand der übermittelten medizinischen Daten erkennen, wie die Qualität der Beatmung oder der Zustand des Patienten bezüglich der Beatmung und/oder Therapie ist und daraufhin entscheiden ob eine weitere, dauerhafte Datenübertragung und damit auch Überwachung des Beatmungsgerätes 1 notwendig ist. Die kürzeren Zeitintervalle zur Verbindung und gegebenenfalls Datenübertragung zwischen Beatmungsgerät 1 und Server 7 kann zum Beispiel dazu dienen, eine dauerhafte Überwachung zu vermeiden, aber stattdessen von Zeit zu Zeit den Zustand des Patienten zu kontrollieren. Beispielsweise ist auch denkbar, dass sich bei gleichbleibend gutem Zustand des Patienten und/oder Nutzung des Beatmungsgerätes 1 die Zeitintervalle zwischen den Verbindungen und eventuellen Datenübertragungen nach und nach verlängern, bis zum Beispiel Zeitintervalle von 1 bis 7 Tagen zwischen den Verbindungen liegen. Bei einer Übertragung eines Datenpaketes, also zum Beispiel einer Zusammenfassung der Daten des Zeitraumes seit der letzten Verbindung, kann beispielsweise ein Zeitintervall von 1 bis 7 Tagen bestimmt werden. So kann zum Beispiel eine tägliche oder auch wöchentliche Zusammenfassung der Daten des Beatmungsgerätes 1 vom Server 7 angefordert und empfangen werden. In manchen Ausführungsformen ist es so zum Beispiel möglich, dass lediglich durch den Server 7 die Datenübertragung auf ordentlichem Weg beendet werden kann. Ein nicht-ordentlicher Weg zur Beendigung der Datenübertragung wäre beispielsweise das Entfernen der Stromversorgung des Beatmungsgerätes 1 oder das erzwungene Deaktivieren der Datenverbindungen des Beatmungsgerätes 1.

Nachdem die Verbindung zwischen Server 7 und Beatmungsgerät 1 getrennt wurde wartet das Beatmungsgerät 1 das vorgegebene Zeitintervall ab und stellt erneut eine Verbindung mit dem Server 7 her. Die Verbindung wird dabei nur dann hergestellt, wenn das Beatmungsgerät 1 dazu in der Lage ist, also zum Beispiel eine Stromversorgung, beispielsweise über die örtliche Energieversorgung, sowie eine Datenverbindung verfügbar ist. Es kann also zwischen verbindungsfähigen Modi und nicht-verbindungsfähigen Modi unterschieden werden. Ein verbindungsfähiger Modus ist beispielsweis, wenn eine ausreichende Stromversorgung vorhanden ist, beispielsweise über eine örtliche Energieversorgung oder einem ausreichend geladenen Akku, und eine Datenverbindung zur Verfügung steht. Ein nicht-verbindungsfähiger Modus liegt zum Beispiel vor, wenn das Beatmungsgerät 1 in einem sogenannten Flugmodus ist, d.h. wenn alle Datenverbindungen deaktiviert sind und nicht zur Verfügung stehen. Ein solcher Flugmodus wird unter anderem während der Therapie eingeschaltet, insbesondere während der Schlaftherapie, damit der Patient nicht zusätzlich durch Funkstrahlen beeinflusst werden kann. Teilweise kann es auch erwünscht sein, eine Datenverbindung während der Therapie zu ermöglichen, beispielsweise für ein "Live"-Monitoring/Echtzeitüberwachung.

Dazu kann beispielsweise eine kabelgebundene Datenverbindung genutzt werden oder auch die drahtlosen Datenverbindungen aktiviert werden. In manchen Ausführungsformen erkennt das Beatmungsgerät 1 zudem automatisch, ob eine ausreichende Stromversorgung vorhanden ist. Beispielsweise können die Datenverbindungen eingeschränkt oder deaktiviert werden, sobald das Beatmungsgerät 1 vom Stromnetz getrennt wird. Zusätzlich oder alternativ ist auch eine Deaktivierung der Datenverbindungen bei niedrigem Akkustand (z.B. unter 50% oder unter 30% oder unter 15%) möglich. Je nach Qualität der Datenverbindung ist es beispielsweise auch denkbar, dass die zu übertragene Datenmenge durch das Beatmungsgerät 1 und/oder den Server 7 angepasst wird, zum Beispiel bei schlechteren Datenverbindungen werden weniger Daten übermittelt. Zudem ist auch eine Priorisierung der zu übertragenen Daten entsprechend der Stromversorgung und der Datenverbindung denkbar.

In manchen Ausführungsformen ist ein Herstellen der Verbindung zwischen dem Beatmungsgerät 1 und dem Server 7 nur durch die Kommunikationseinheit 3 vorgesehen. Somit kann eine Verbindung nur aufgebaut werden, wenn durch das Beatmungsgerät 1 oder einem Nutzer/Anwender/Patienten am Beatmungsgerät 1 ein Verbindungsaufbau angestrebt wird.

Bei jeder neu hergestellten Verbindung zum Server 7 sendet das Beatmungsgerät 1 zunächst immer Basisinformationen/Geräteinformationen, wie zum Beispiel den Gerätetyp, Seriennummer, Firmware-Version und gegebenenfalls andere Geräteinformationen, an den Server 7. Zusätzlich zu dem beschriebenen Schritt zur Identifikation des Beatmungsgeräts 1 können beispielsweise auch noch weitere Verfahren zur Authentifizierung oder Sicherung der Verbindung durchgeführt werden, bevor mit der weiteren Datenübertragung zwischen Server 7 und Beatmungsgerät 1 begonnen wird. Diese zusätzlichen Authentifizierungs- und Sicherungsschritte können in manchen Ausführungsformen auch vor der Übertragung der Basisinformationen des Beatmungsgerätes 1 an den Server 7 erfolgen.

Der Server 7 kann anhand der übertragenen Basisinformationen das Beatmungsgerät 1 erkennen/identifizieren und individuell entscheiden, ob und welche Art von Datenübertragung angefordert wird sowie welche Daten im Einzelnen vom Beatmungsgerät 1 an den Server 7 übertragen werden sollen. Dazu ist auf dem Server 7 beispielsweise für jedes (dem Server 7 bekannten) individuelle Beatmungsgerät abgespeichert, welche Daten angefordert werden sollen, welche Konfigurationen an das Beatmungsgerät gesendet werden sollen und auch in welchen Zeitintervallen das Beatmungsgerät sich mit dem Server 7 verbinden soll. Der Server 7 gleicht also das identifizierte Beatmungsgerät 1 mit den gespeicherten Beatmungsgeräten ab und sendet die entsprechenden Konfigurationsdaten sowie eine eventuelle Anforderung zur Übertragung von Daten vom Beatmungsgerät 1 an das Beatmungsgerät 1. Entsprechend der Anforderung beginnt das Beatmungsgerät 1 mit der Datenübertragung an den Server 7 zumindest so lange, bis die Übertragung vom Server 7 beendet wird. Während der Datenübertragung können zudem auch beispielsweise medizinische und/oder technische Konfigurationsdaten und/oder Konfigurationsdaten zur Kommunikation an das Beatmungsgerät 1 übermittelt werden. Medizinische Konfigurationsdaten enthalten beispielsweise Einstellwerte zur Therapie wie z.B. Beatmungsdrücke, Flussraten, Dauer und andere. Unter technischen Konfigurationsdaten können zum Beispiel Daten gezählt werden, welche die technische Funktion des Beatmungsgerätes 1 an sich betreffen, wie beispielsweise ein Firmware-Update. Die Konfigurationsdaten zur Kommunikation enthalten zum Beispiel Einstellungen zur zu übertragenden Datenmenge, Datenarten und/oder Zeitintervallen in denen sich das Beatmungsgerät 1 mit dem Server 7 verbinden soll. In manchen Ausführungsformen überträgt der Server 7 zumindest immer das Zeitintervall nach welchem sich das Beatmungsgerät 1 wieder mit dem Server 7 verbinden soll.

Die Datenabfrage durch den Server 7, also das Anfordern einer Datenübertragung von medizinischen Daten des Beatmungsgerätes 1 kann in manchen Ausführungsformen auch automatisch durch den Server 7 erfolgen. Beispielsweise kann der Server 7 automatisch bei jeder Verbindung des Beatmungsgerätes 1 zum Server 7 nach einem Zeitintervall medizinische Daten vom Beatmungsgerät 1 anfordern, ohne, dass dies zuvor von einem Nutzer am Server 7 explizit festgelegt werden muss. Wie beschrieben, kann der Server 7 anhand der übertragenen medizinischen Daten auch automatisch festlegen, ob weitere medizinische Daten übertragen werden und/oder wann die nächste Datenübertragung erfolgen soll. Fordert der Server 7 keine medizinischen Daten vom Beatmungsgerät 1 an, so werden medizinische Daten auch nicht vom Beatmungsgerät 1 an den Server 7 übertragen. Während in manchen Ausführungsformen der Server 7 vollständig automatisch über die Übertragung von medizinischen Daten entscheidet, ist es in manchen Ausführungsformen vorgesehen, dass ein Nutzer des Servers 7 zumindest einzelne Datenübertragungen manuell anfordern kann.

In manchen beispielhaften Ausführungsformen wird die Datenübertragung zwischen Server 7 und Beatmungsgerät 1 anhand einer Eingabe am Server 7 und/oder einer Eingabe am Beatmungsgerät 1 beendet. Es ist zudem denkbar, dass sowohl durch Eingaben am Server 7 als auch durch Eingaben am Beatmungsgerät 1 eine Verbindung unabhängig von dem eingestellten Zeitintervall hergestellt wird. In manchen Ausführungsformen kann das Beatmungsgerät 1 auch automatisch unabhängig von dem eingestellten Zeitintervall eine Verbindung zum Server 7 herstellen und mit der Datenübertragung beginnen. Dies kann beispielsweise der Fall sein, wenn die durch die Sensoreinheit 62 aufgenommenen und durch die Aufbereitungseinheit 63 ausgewerteten Messwerte und Daten eine Verschlechterung des Zustandes des Patienten und/oder der Qualität der Beatmung erfasst wird. Die kann beispielsweise durch die Über- und/oder Unterschreitung von Schwellenwerten mit Bezug auf die Atemfrequenz, Drücke und/oder Flüsse und/oder Leckage und/oder Therapieadhärenz und/oder eine Verschlechterung der Synchronität zwischen Gerät und Patient und/oder Erkennung eines intrinsischen PEEPs des Patienten und/oder Veränderung von CO2, SpO2, Schlafqualität, Temperatur oder Aktivität des Patienten erkannt werden. Auch kann der Server 7 anhand einer Analyse der vom Beatmungsgerät 1 übertragenen medizinischen Daten automatisch die Zeitintervalle, in denen eine Verbindung vom Beatmungsgerät 1 zum Server 7 hergestellt werden soll, anpassen. Beispielsweise kann der Server 7 eine im Wesentlichen gleichbleibende Qualität der Beatmung so bewerten, dass die Zeitintervalle, nach denen vom Beatmungsgerät 1 eine Verbindung zum Server 7 hergestellt und eine Datenübertragung durch den Server 7 angefordert wird, vergrößert werden. Eine gleichbleibende Qualität der Beatmung kann der Server 7 etwa anhand der vom Beatmungsgerät 1 übertragenen medizinischen Daten erkennen. Eine gleichbleibende Qualität der Beatmung kann zum Beispiel erkannt werden, wenn die mit der Atmung zusammenhängenden Parameter wie Druck, Fluss und/oder Frequenz im Wesentlichen keine Schwankungen aufweisen und/oder auch keine Über- bzw. Unterschreitung von Schwellenwerten aufweisen. Werden andererseits Schwankungen oder Abweichungen bei mit der Atmung zusammenhängenden Parametern und/oder eine Über- bzw. Unterschreitung von Schwellenwerten erkannt werden, so kann neben der entsprechenden Anpassung der Beatmungsparameter durch die Steuereinheit 4 auch durch den Server 7 eine Verkürzung der Zeitintervalle vorgegeben werden. Zum einen ist es denkbar, dass das Beatmungsgerät 1 die Daten bereits durch die Aufbereitungseinheit 63 ausgewertet, sodass die Anhaltspunkte zur Qualität der Beatmung direkt an den Server 7 gesendet werden und der Server 7 anhand dieser Anhaltspunkte über die weiteren Datenübertragungen entscheiden kann. Zum anderen ist es beispielsweise auch möglich, dass der Server 7 selber die medizinischen Daten des Beatmungsgerätes 1 hinsichtlich der Beatmungsqualität bewertet und daraufhin über die weiteren Datenübertragungen und Verbindungen entscheidet.

Wird nach dem eingestellten Zeitintervall keine Verbindung zwischen Beatmungsgerät 1 und Server 7 hergestellt, so wird dies von sowohl dem Beatmungsgerät 1 als auch dem Server 7 registriert. Nach einer bestimmten Anzahl von nicht erfolgten Verbindungen kann durch den Server 7 und/oder das Beatmungsgerät 1 eine Alarmmeldung generiert werden, welche auf die fehlgeschlagenen Verbindungen hinweist. Dazu ist auch ein durch den Server 7 initiierter Verbindungsversuch zu dem Beatmungsgerät 1 denkbar, wenn über einen längeren Zeitraum keine Verbindung aufgebaut wurde.

In manchen Ausführungsformen verkürzt das Beatmungsgerät 1 das Zeitintervall bis zur nächsten Verbindung mit dem Server 7 automatisch, falls nach dem regulären Zeitintervall keine Verbindung hergestellt werden konnte. Beträgt das ursprüngliche Zeitintervall zum Beispiel 24 Stunden, um zum Beispiel eine Nutzungszusammenfassung des vergangenen Tages an den Server zu übermitteln, schlägt die Verbindung zum Server 7 nach 24 Stunden aber fehl, versucht das Beatmungsgerät 1 zum Beispiel nach einem verkürzten Zeitintervall von 1 Stunde erneut eine Verbindung aufzubauen, bevor nach einer bestimmten Anzahl von Fehlversuchen eine Alarmmeldung generiert wird.

Findet keine dauerhafte Datenübertragung, also ein "Live"-Monitoring bzw. eine Echtzeitüberwachung, statt, so werden die von der Sensoreinheit 62 in der Überwachungseinheit 65 ermittelten Werte und Daten und von der Aufbereitungseinheit 63 verarbeiteten Daten in der Speichereinheit 64 zumindest zwischengespeichert und, falls vom Server 7 angefordert, mit der nächsten Datenübertragung an den Server übertragen.

In einigen beispielhaften Ausführungsformen findet eine Übertragung von medizinischen Daten insbesondere nur dann statt, wenn diese Daten explizit durch den Server 7 angefordert werden.

Es ist auch denkbar, dass die Übertragung dauerhaft oder auch für immer beendet wird. Dies ist von Vorteil, wenn das Beatmungsgerät 1 beispielsweise den Anwender wechselt und neu konfiguriert bzw. eingestellt wird In diesem Fall kann es vorteilhaft sein, das Gerät 1 vom Server 7 dauerhaft abzumelden. Der Server 7 kann das Beatmungsgerät 1 dauerhaft deaktivieren. Es ist beispielsweise denkbar, dass der Server 7 eine vom Gerät 1 umfasste SIM-Karte im Gerät 1 deaktiviert.

### Bezugszeichenliste

- 1: Beatmungsgerät
- 2: Eingabeeinheit
- 3: Kommunikationseinheit
- 4: Steuereinheit
- 5: Benutzerschnittstelle
- 7: Server
- 61: Gebläse-/Ventileinheit
- 62: Sensoreinheit
- 63: Aufbereitungseinheit
- 64: Speichereinheit
- 65: Überwachungseinheit

## Patentansprüche

1. Verfahren zur Datenübertragung innerhalb eines Systems, wobei das System zumindest ein Beatmungsgerät (1) und einen Server (7) umfasst, wobei das Beatmungsgerät (1) zumindest eine Eingabeeinheit (2) und zumindest eine Kommunikationseinheit (3) umfasst, wobei die Eingabeeinheit (2) eingerichtet und ausgebildet ist, eingegebene Werte und Informationen dem System bereitzustellen und wobei die Kommunikationseinheit (3) eingerichtet und ausgebildet ist, zumindest eine Verbindung zu zumindest einem Server (7) herzustellen und Daten zwischen dem Server (7) und dem Beatmungsgerät (1) zu übertragen, also an den Server (7) zu senden und vom Server (7) zu empfangen, **dadurch gekennzeichnet, dass** die Datenübertragung durch den Server (7) beendet wird, wobei während der Datenübertragung vom Server (7) an das Beatmungsgerät (1) Konfigurationsdaten gesendet, in denen medizinische Konfigurationsdaten, technische Konfigurationsdaten und Konfigurationsdaten zur Kommunikation enthalten sind, wobei die Konfigurationsdaten zur Kommunikation Einstellungen zur Datenmenge, die das Beatmungsgerät während der Datenübertragung an den Server senden soll, sowie auch zum Zeitintervall enthalten.

2. Verfahren nach Anspruch 1, **dadurch gekennzeichnet, dass** mittels der Konfiguration zur Kommunikation auch eingestellt wird, wie detailliert die medizinischen und technischen Daten des Beatmungsgerätes an den Server übermittelt werden sollen, indem die medizinischen Daten in unterschiedlichen Detailgraden gesendet werden, wobei der Detailgrad der Übermittlung vom Server (7) vorgeben wird oder von einem Benutzer des Servers aus der Ferne eingestellt wird.

3. Verfahren nach zumindest einem der vorhergehenden Ansprüche, **dadurch gekennzeichnet, dass** das Beatmungsgerät (1) bei jeder neu hergestellten Verbindung zum Server (7) zunächst Geräteinformationen, welche Informationen wie Gerätetyp, Seriennummer, Firmware-Version enthalten, an den Server sendet, wobei der Server anhand dieser Informationen zunächst das Beatmungsgerät (1) erkennt und daraufhin individuell für dieses Beatmungsgerät (1) entscheidet, ob und welche Datenmenge vom Beatmungsgerät (1) an den Server gesendet werden soll, wobei diese Entscheidung als Konfiguration zur Kommunikation vom Server an das Beatmungsgerät gesendet wird und basierend auf dieser Konfiguration zur Kommunikation das Beatmungsgerät mit der Datenübertragung beginnt.

4. Verfahren nach zumindest einem der vorhergehenden Ansprüche, **dadurch gekennzeichnet, dass** der Server (7) anhand der Geräteinformationen des Beatmungsgerätes (1) entscheidet, dass eine neue Firmware-Version an das Beatmungsgerät (1) gesendet wird.

5. Verfahren nach zumindest einem der vorhergehenden Ansprüche, **dadurch gekennzeichnet, dass** als medizinische Daten die Nutzungszeiten und -dauer des Beatmungsgerätes (1) an den Server (7) übertragen werden.

6. Verfahren nach zumindest einem der vorhergehenden Ansprüche, **dadurch gekennzeichnet, dass** bevor der Server (7) die Verbindung zum Beatmungsgerät (1) trennt, zumindest Konfigurationsdaten zur Kommunikation übermittelt werden, welche zumindest das Zeitintervall vorgeben, nach welchem das Beatmungsgerät (1) die nächste Verbindung zum Server (7) herstellen soll.

7. Verfahren nach zumindest einem der vorhergehenden Ansprüche, **dadurch gekennzeichnet, dass** nachdem die Verbindung zwischen Server (7) und Beatmungsgerät (1) getrennt wurde das Beatmungsgerät (1) das vorgegebene Zeitintervall abwartet und dann erneut eine Verbindung mit dem Server (7) herstellt.

8. Verfahren nach zumindest einem der vorhergehenden Ansprüche, **dadurch gekennzeichnet, dass** der Server (7) eine im Wesentlichen gleichbleibende Qualität der Beatmung bewertet, wobei die Zeitintervalle, nach denen vom Beatmungsgerät (1) eine Verbindung zum Server (7) hergestellt und eine Datenübertragung durch den Server (7) angefordert wird, vergrößert werden, wobei eine gleichbleibende Qualität der Beatmung anhand der vom Beatmungsgerät (1) übertragenen medizinischen Daten erkannt wird, wenn die mit der Atmung zusammenhängenden Parameter wie Druck, Fluss und/oder Frequenz im Wesentlichen keine Schwankungen aufweisen und/oder auch keine Über- oder Unterschreitung von Schwellenwerten aufweisen.

9. Verfahren nach zumindest einem der vorhergehenden Ansprüche, **dadurch gekennzeichnet, dass** wenn Schwankungen oder Abweichungen bei mit der Atmung zusammenhängenden Parametern und/oder eine Über- oder Unterschreitung von Schwellenwerten erkannt werden, neben der entsprechenden Anpassung der Beatmungsparameter durch eine Steuereinheit (4) auch durch den Server (7) eine Verkürzung der Zeitintervalle vorgegeben wird.

10. Verfahren nach zumindest einem der vorhergehenden Ansprüche, **dadurch gekennzeichnet, dass** das Beatmungsgerät (1) die Daten bereits durch eine Aufbereitungseinheit (63) auswertet, sodass Anhaltspunkte zur Qualität der Beatmung direkt an den Server (7) gesendet werden und der Server anhand dieser Anhaltspunkte über die weiteren Datenübertragungen entscheiden kann, wobei der Server selber die medizinischen Daten des Beatmungsgerätes hinsichtlich der Beatmungsqualität bewertet und daraufhin über die weiteren Datenübertragungen und Verbindungen entscheidet.

11. Verfahren nach zumindest einem der vorhergehenden Ansprüche, **dadurch gekennzeichnet, dass** die Übertragung dauerhaft beendet wird, indem das Beatmungsgerät (1) vom Server (7) dauerhaft abgemeldet wird, indem der Server 7 eine vom Gerät 1 umfasste SIM-Karte im Beatmungsgerät deaktiviert.

12. Verfahren nach zumindest einem der vorhergehenden Ansprüche, **dadurch gekennzeichnet, dass** wenn die Datenübertragung zwischen Beatmungsgerät und Server beendet wurde und damit einhergehend auch die Verbindung zwischen Beatmungsgerät (1) und Server (7) getrennt ist, so ist für das Beatmungsgerät eingestellt, dass bei getrennter Verbindung zwischen Beatmungsgerät und Server in einem bestimmten Zeitintervall, welches am Beatmungsgerät festgelegt wird und/oder durch den Server vorgegeben wird, durch die Kommunikationseinheit eine Verbindung zum Server hergestellt wird beziehungsweise ein Verbindungsversuch erfolgt, wobei die Verbindung nur dann hergestellt, wenn das Beatmungsgerät zu einer Verbindung und Datenübertragung über Mobilfunk in der Lage ist.

13. Verfahren nach zumindest einem der vorhergehenden Ansprüche, **dadurch gekennzeichnet, dass** die zu übertragene Datenmenge je nach Qualität der Datenverbindung durch das Beatmungsgerät (1) und/oder den Server (7) angepasst wird.

14. System mit zumindest ein Beatmungsgerät (1) und einen Server (7), wobei das Beatmungsgerät (1) zumindest eine Eingabeeinheit (2) und zumindest eine Kommunikationseinheit (3) umfasst, wobei die Eingabeeinheit (2) eingerichtet und ausgebildet ist, eingegebene Werte und Informationen dem System bereitzustellen und wobei die Kommunikationseinheit (3) eingerichtet und ausgebildet ist, zumindest eine Verbindung zu zumindest einem Server (7) herzustellen und Daten zwischen dem Server (7) und dem Beatmungsgerät (1) zu übertragen, also an den Server (7) zu senden und vom Server (7) zu empfangen, **dadurch gekennzeichnet, dass** die Datenübertragung durch den Server (7) beendet wird.

15. Beatmungsgerät (1) **dadurch gekennzeichnet, dass** das Beatmungsgerät in einem Verfahren oder einem System nach zumindest einem der vorhergehenden Ansprüchen verwendet wird.
